# EUROPÄISCHE PATENTSCHRIFT

(11) **EP 2 970 108 B1**
(45) Veröffentlichungstag und Bekanntmachung des Hinweises auf die Patenterteilung: **27.06.2018**
(21) Anmeldenummer: 14708281.2
(22) Anmeldetag: 07.03.2014
(51) Int. Cl.: C07C 269/02, C08G 18/80, C08G 18/10

(54) **VERFAHREN ZUR HERSTELLUNG VON FARBHELLEN TDI-POLYISOCYANATEN**
PROCESS FOR THE PREPARATION OF LIGHTLY COLOURED TDI POLYISOCYANATES
PROCÉDÉ DE FABRICATION DE POLYISOCYANATES DE TDI DE COULEUR CLAIRE

(30) Priorität: 12.03.2013 EP 13158689
(43) Veröffentlichungstag der Anmeldung: 20.01.2016
(73) Patentinhaber: Covestro Deutschland AG, 51373 Leverkusen (DE)
(72) Erfinder: SANDERS, Josef, 51375 Leverkusen (DE); HECKING, Andreas, 40764 Langenfeld (DE); HALPAAP, Reinhard, 51519 Odenthal (DE); RICHTER, Frank, 51373 Leverkusen (DE); WILMES, Oswald, 51061 Köln (DE); BUSCH, Jan, 40477 Düsseldorf (DE); LODDENKEMPER, Tim, 41542 Dormagen (DE); STEFFENS, Friedhelm, 51373 Leverkusen (DE)
(74) Vertreter: Levpat
(86) Internationale Anmeldenummer: PCT/EP2014/054423
(87) Internationale Veröffentlichungsnummer: WO 2014/139873

(56) Entgegenhaltungen:
- EP-A1- 1 413 571
- EP-A2- 0 546 399
- HACH Lange GmbH: "objective Color Assessment and Quality Control in Chemical, PharmaceuticalCosmetic Industries", 2016

## Beschreibung

Die Erfindung betrifft Urethangruppen aufweisende Polyisocyanate auf Basis von Toluylendiisocyanat, ein Verfahren zu ihrer Herstellung und ihre Verwendung als Polyisocyanatkomponente in Ein- und Zweikomponenten-Polyurethanlacken.

Urethangruppen aufweisende Polyisocyanate aus niedermolekularen Polyhydroxyverbindungen und Toluylendiisocyanat (TDI) sind seit langem bekannt und werden z.B. in den deutschen Patentschriften DE 870 400, 953 012 und DE 1 090 196 beschrieben. Derartige Produkte sind von großer Bedeutung im Bereich der Polyurethanlacke und -beschichtungen, insbesondere in der Holzlackierung, sowie im Klebstoffsektor. Handelsübliche Produkte werden heutzutage so hergestellt, dass Polyhydroxyverbindungen mit der 5- bis 10-fachen molaren Menge Toluylendiisocyanat umgesetzt werden, worauf sich eine destillative Entfernung des überschüssigen Ausgangsdiisocyanats, vorzugsweise im Dünnschichtverdampfer, anschließt. Derartige Verfahren sind beispielsweise in DE-PS 1 090 186 bzw. in der US-PS 3,183,112 beschrieben. In der EP 0 546 399 werden Ether und Urethangruppen aufweisende Polyisocyanate auf Basis von Polyhydroxypolyethern und Toluylendiisocyanat mit einem Gehalt an freiem TDI von <0,1% beansprucht. Die auf diese Weise hergestellten Polyisocyanate des Standes der Technik weisen jedoch den grundlegenden Nachteil auf, dass sie wie die meisten aromatischen Polyisocyanate eine im Vergleich zu aliphatischen Lackpolyisocyanaten deutlich höhere Eigenfarbe aufweisen. Aufgrund dieser Eigenfarbe sind sie insbesondere für die Lackierung von in jüngster Zeit stärker nachgefragten helleren Holzarten nur eingeschränkt einsetzbar, da die damit lackierten Teile dunkler erscheinen und ihre schon vor der Lackierung nur dezente Maserung nach der Lackierung kaum noch erkennbar ist.

Sowohl die Eigenfarbe des eingesetzten TDIs als auch eine nicht ausreichende Reinheit von farbhellem TDI führen häufig zu verfärbten Polyisocyanaten. Es hat daher nicht an Versuchen gefehlt, TDI-Qualitäten bereitzustellen, mit denen farbhellere aromatische Polyisocyanate hergestellt werden können. Beispielsweise wird in der EP 1 413 571 ein Verfahren beschrieben, mit dem durch Vorkonzentrierung der TDI-Rohlösung auf einen Lösungsmittelgehalt von <20% und anschließende Fraktionierung in einer Trennwanddestillationskolonne eine Produktfraktion mit einem TDI-Gehalt von mindestens 99,5% und weniger als 200 Gew.-ppm Lösungsmittel und/oder chlorierte aromatische Kohlenwasserstoffe, weniger als 100 Gew.-ppm hydrolisierbares Chlor und weniger als 40 Gew.-ppm Säure erhalten wird. In US-PS 6,900,348 bzw. in der entsprechenden EP 1 187 808 wird beschrieben, dass durch Verwendung von Phosgen mit einem Brom-Gehalt von <50 ppm farbhellere Diphenylmethan-diisocyanate erhalten werden können. Die EP 0 816 333 beansprucht ein Verfahren zur Verringerung der Farbe von TDI durch Behandlung der Rohlösung mit Wasserstoff vor der Abtrennung des Lösungsmittels.

Auch eine spezielle Vorbehandlung des zur Herstellung von TDI verwendeten Toluylendiamins (TDA) kann zu Verringerung der TDI-Farbe führen. Beispielsweise beansprucht die EP 1 864 969 ein Verfahren zur Herstellung von farbhellerem TDI, bei dem das hierfür in die Phosgenierung eingesetzte TDA weniger als 0,1 Gew.-% alkylierte cyclische Ketone bezogen auf 100 Gew.-% TDA enthält. In der US-PS 5,872,278 bzw. der entsprechenden EP 0 866 057 wird ein Verfahren beschrieben, bei dem das eingesetzte Amin vor der Umsetzung mit Phosgen mit Feststoffen behandelt wird, die Lewis- und/oder Brönstedt-Säure-Zentren enthalten. Die erhaltenen Isocyanate weisen dann eine hellere Farbe auf, als Isocyanate, die mit unbehandeltem Amin hergestellt wurden.

Mit diesen vergleichsweise sehr aufwändigen Verfahren können zwar TDI-Qualitäten mit größerer Reinheit und hellerer Farbe hergestellt werden, jedoch findet sich dort kein Hinweis darauf, welche Nebenkomponenten für die immer noch nicht ausreichend zu verhindernde Verfärbung der Polyisocyanate bei ihrer Herstellung verantwortlich sind und wie diese Verfärbung in ausreichendem Maß verhindert werden kann. Es besteht daher immer noch ein dringender Bedarf an farbhellen aromatischen Lackpolyisocyanaten.

Aufgabe der vorliegenden Erfindung war es ein Verfahren zu finden, mit dem farbhelle Urethangruppen aufweisende Polyisocyanate auf Basis von Toluylendiisocyanat hergestellt werden können.

Diese Aufgabe konnte mit dem nachstehend näher beschriebenen Verfahren gelöst werden.

Der Erfindung liegt die überraschende Beobachtung zugrunde, dass farbhelle Polyisocyanate auf Basis von Toluylendiisocyanat hergestellt werden können, indem man niedermolekulare Polyhydroxyverbindungen mit der 5- bis 10-fachen Menge TDI umsetzt und anschließend das überschüssige TDI vorzugsweise durch Dünnschichtdestillation im Vakuum entfernt, wobei das eingesetzte TDI einen Gehalt an 2-Chloro-6-isocyanato-methylcyclohexadienen (CIMCH) von <5 Gew.-ppm aufweist. Farbhell in diesem Zusammenhang bedeutet, dass die solcherart hergestellten Polyisocyanate APHA-Farbzahlen von <50 Hazen bevorzugt <30 Hazen, besonders bevorzugt ≤ 25 Hazen, gemessen auf Basis der DIN EN 1557, aufweisen.

CIMCH kann in Form von 3 Doppelbindungsisomeren vorliegen, die im TDI in unterschiedlichem Verhältnis enthalten sein können. Diese werden z. B. bei der TDI-Herstellung aus im eingesetzten TDA enthaltenen 1-Amino-2-methyl-cyclohexenon gebildet, das wiederum bei der Herstellung von TDA aus Dinitrotoluol (DNT) durch partielle Kernhydrierung von TDA und Ersatz einer Aminofunktion durch Wasser entstehen kann. Es ist auch möglich, dass die Ketofunktion bereits anteilig durch oxidativen Angriff bei der Herstellung von DNT durch Nitrierung von Toluol eingeführt wird, wobei zunächst Nitrokresole entstehen, die dann bei der anschließenden Hydrierung das oben beschriebene 1-Amino-methyl-2-cyclohexenon bilden können.

Gegenstand der Erfindung ist somit ein Verfahren zur Herstellung von farbhellen Polyisocyanaten mit APHA-Farbzahlen <50 Hazen, durch Umsetzung von organischen Polyhydroxyverbindungen mit überschüssigen Mengen an Toluylendiisocyanat und anschließender destillativer Entfernung von nicht umgesetzten Toluylendiisocyanat, dadurch gekennzeichnet, dass das eingesetzte Toluylendiisocyanat einen Gehalt an 2-Chloro-6-isocyanato-methylcyclohexadienen (CIMCH) von <5 Gew.-ppm aufweist.

Gegenstand der Erfindung sind auch die nach dem erfindungsgemäßen Verfahren erhältlichen farbhellen Polyisocyanate mit APHA-Farbzahlen <50 Hazen, sowie deren Verwendung als Polyisocyanatkomponente in Polyurethanlacken, insbesondere in Zweikomponenten-Polyurethanlacken.

Ausgangsmaterialien für das erfindungsgemäße Verfahren sind Toluylendiisocyanat und niedermolekulare Polyhydroxyverbindungen.

Als Toluylendiisocyanat kommen insbesondere 2,4-Toluylendiisocyanat und seine technischen Gemische mit bis zu 35 Gew.-%, bezogen auf Gemisch, an 2,6-Toluylendiisocyanat in Betracht, die einen Gehalt an 2-Chloro-6-isocyanato-methylcyclohexadienen (CIMCH) von <5 Gew.-ppm, bevorzugt von ≤ 3 Gew.-ppm aufweisen. Derartige TDI-Qualitäten können beispielsweise durch gezielte destillative Entfernung von 2-Chloro-6-isocyanato-methylcyclohexadienen aus den vorkonzentrierten TDI-Rohlösungen mittels einer Trennwanddestillationskolonne erhalten werden, wie dies in der EP 1 413 571 B1 beschrieben ist. Besonders bevorzugt sind jedoch Toluylendiisocyanate, die durch Gasphasenphosgenierung von TDA hergestellt werden und deren Gehalt an 2-Chloro-6-isocyanato-methylcyclohexadienen unterhalb der Nachweisgrenze liegt. Toluylendiisocyanat einer derartigen Qualität ist z.B. von der Bayer Material Science AG aus der Produktion am Standort Caojing in China erhältlich.

Zur eindeutigen Charakterisierung der Komponente 2-Chloro-6-isocyanato-methylcyclohexadienen wurden zwei voneinander unabhängige analytische Methoden genutzt. Mittels gaschromatographischer Techniken wurden unterschiedliche Toluylendiisocyanat-Qualitäten mit einem 2,4-Anteil von rund 80 Gew.-% auf ihre Ungleichheiten in dem Nebenkomponentenspektrum hin untersucht. Durch anschließende gekoppelte gaschromatographische Massenspektroskopie wurde den bis dahin drei unbekannten Verbindungen (CIMICH einschließlich zweier Isomeren) ein Molekulargewicht von 169g/mol zugeordnet. Weitere Strukturinformationen konnten durch dem Fachmann bekannte Weise aus der Fragmentierung gewonnen werden. Mittels aufwendigen kernresonanzspektroskopischen Experimenten (¹H-NMR, ¹H-COSY, ¹H-, ¹H-TOCSY und ¹H-, ¹³C-HMBC) konnten den drei Komponenten mit m/z 169 die unten aufgeführten Strukturen zugeordnet werden.

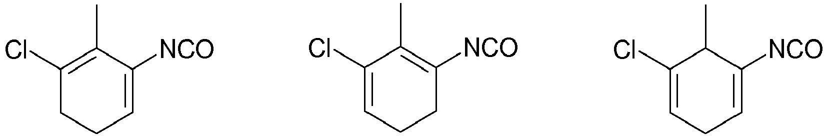

Durch gezielte Methodenentwicklung konnte die Nachweisgrenze der Isomeren des CIMICH mittels gaschromatographischer Spektroskopie, unter Verwendung einer Optima 5 HT Säule (60m Länge, 0,25mm Innendurchmesser, 0,25µm Filmdicke) von Macherey-Nagel in einem HP Series 6890 Gaschromatographen von Hewlett Packard auf 1 Gew.-ppm festgelegt werden.

Als beim erfindungsgemäßen Verfahren einzusetzende niedermolekulare Polyhydroxyverbindungen kommen zwei- bis vierwertige Alkohole mit einem Molekulargewicht von 62 bis 146 und/oder aus ihnen durch Addition von Ethylen- und/oder Propylenoxid hergestellte Polyetherpolyole in reiner Form oder als beliebige Gemische zum Einsatz.

Als zwei- bis vierwertige Alkohole kommen zum Beispiel in Betracht Ethylenglykol, 1,2-Propylenglykol, 1,3-Propylenglykol, 1,2-Butandiol ,1,3-Butandiol, 1,4-Butandiol, 1,5-Pentandiol, Neopentylglykol, 1,6-Hexandiol, 2-Ethylhexandiol, Glycerin, Trimethylolpropan und Pentaerythrit.

Geeignete Polyetherpolyole weisen ein aus Hydroxylgruppengehalt und Hydroxylfunktionalität berechenbares Molekulargewicht von 106 bis 600, vorzugsweise 106 bis 470 auf. Bevorzugt werden Polyetherdiole und Polyethertriole eingesetzt. Diese Polyetherpolyole können in an sich bekannter Weise durch Alkoxylierung geeigneter zwei- bis vierfunktioneller Startermoleküle oder geeigneter Gemische von Startermolekülen erhalten werden, wobei bei der Alkoxylierung insbesondere Propylenoxid und/oder Ethylenoxid, gegebenenfalls im Gemisch nacheinander in beliebiger Reihenfolge zum Einsatz gelangen. Bevorzugt werden als Startermoleküle die oben genannnten zwei- bis vierwertigen Alkohole eingesetzt. Ganz besonders bevorzugt werden Gemische aus Trimethylolpropan und Diethylenglykol eingesetzt.

Zur Durchführung des erfindungsgemäßen Verfahrens werden die Polyhydroxyverbindungen mit Toluylendiisocyanat bei Temperaturen von 40 bis 140°C, vorzugsweise 70 bis 110°C umgesetzt. Die Mengen der Reaktionspartner entsprechen dabei im Allgemeinen einem NCO/OH-Äquivalentverhältnis von 4:1 bis 20:1, vorzugsweise 5:1 bis 10:1.

Nach der Prepolymerisierung wird der Überschuss an nicht umgesetztem Toluylendiisocyanat durch Vakuum-Dünnschichtdestillation bei 100 bis 180°C, vorzugsweise 120 bis 160°C entfernt. Dabei erhält man die erfindungsgemäßen Polyisocyanate in Form harter bis halbharter Harze.

Die nach dem erfindungsgemäßen Verfahren hergestellten Polyisocyanate stellen Polyisocyanatgemische dar, deren gelchromatographische Analyse das gleichzeitige Vorliegen mehrerer Polyisocyanatkomponenten anzeigt, wobei deren Zusammensetzung und Funktionalität von den eingesetzten Hydoxyverbindungen sowie dem bei der Prepolymerisierung vorliegenden TDI-Überschuß abhängt. Hauptprodukte sind jeweils die "monomeren" Urethane die durch Umsetzung der einzelnen Hydroxyverbindungen mit der ihrer Funktionalität entsprechenden Anzahl von TDI-Molekülen entstanden sind. Zusätzlich sind aber auch oligomere Urethane mit höheren Molgewichten enthalten, die zwei oder mehrere Polyhydroxy-Moleküle enthalten können, wobei der relative Anteil der höhermolekularen Oligomeren mit zunehmendem TDI-Überschuss bei der Prepolymerisierung sinkt. Die Funktionalitäten der Hauptkomponenten entsprechen denen der eingesetzten Polyhydroxykomponenten und liegen demnach bei zwei, drei oder vier, während die als Nebenkomponenten enthaltenen Oligomeren jeweils höherere Funktionalitäten aufweisen. Die aus Isocyanatgehalt und gelchromatographisch bestimmtem Molekulargewicht errechenbare mittlere Funktionalität der nach dem erfindungsgemäßen Verfahren hergestellten Produkte liegt zwischen 2,0 bis 4,0, bevorzugt 2,5 bis 3,5, ganz besonders bevorzugt zwischen 2,8 bis 3,2. Die gelchromatographischen Bestimmungen wurden mit einem Gelchromatographen des Typs HP 1100 von Hewlett Packard durchgeführt. Dazu wurde ein 4-fach Säulensatz von Macherey & Nagel (2 Nucleogel-100 und 2 Nucleogel-50 plus einer Nucleogel-10 Vorsäule) bei 35°C unter Verwendung eines Polystyrolstandards und THF als Elutionsmittel genutzt.

Zur Anwendung gelangen die nach dem erfindungsgemäßen Verfahren hergestellten Polyisocyanate im Allgemeinen in Form einer Lösung in einem organischen Lösemittel. Geeignete Lösemittel sind z.B. die bekannten Lacklösemittel wie Ethylacetat, Butylacetat, Methoxypropylacetat, Toluol, die isomeren Xylole sowie technische Losemittel wie ®Solvent Naphtha oder ®Solvesso oder Gemische derartiger Lösungsmittel.

Die nach dem erfindungsgemäßen Verfahren hergestellten Polyisocyanate stellen wertvolle Rohstoffe für Ein- und Zweikomponenten Polyurethanlacke dar. Das besonders bevorzugte Einsatzgebiet für diese Polyisocyanate ist ihre Verwendung als Polyisocyanatkomponente in Zweikomponenten-Polyurethanlacken. Die für diese bevorzugte Verwendung bevorzugten Reaktionspartner für die erfindungsgemäßen Polyisocyanate sind die in der Polyurethanlacktechnik an sich bekannten Polyhydroxypolyester und -ether, Polyhydroxypolyacrylate und gegebenenfalls niedermolekulare mehrwertige Alkohole.

Auch Polyamine, insbesondere in blockierter Form als Polyketimine oder Oxazolidine sind denkbare Reaktionspartner für das erfindungsgemäße Verfahren. Die Mengenverhältnisse, in welchen die nach dem erfindungsgemäßen Verfahren hergestellten Polyisocyanate und die genannten Reaktionspartner bei der Herstellung von Zweikomponenten-Polyurethanlacken eingesetzt werden, werden im allgemeinen so gewählt, dass auf eine Isocyanatgruppe 0,8 bis 3,0, vorzugsweise 0,9 bis 1,1 gegenüber Isocyanatgruppen reaktionsfähige Gruppen entfallen.

Je nach Wahl der eingesetzten Polyhydroxy-Komponenten können die Zweikomponenten-Polyurethan-Beschichtungsmittel unterschiedliche Topfzeit aufweisen. Falls eine besonders schnelle Durchhärtung gewünscht wird, können die in der Isocyanatchemie üblichen Katalysatoren mitverwendet werden, wie z.B. Amine wie Triethylamin, Pyridin, Methylpyridin, Benzyldimethylamin, N,N'- Dimethylpiperazin oder Metallsalze wie Eisen(III)chlorid, Zinkchlorid, Zink-2-ethylcaproat, Zinn(II)-2-ethylcaproat, Dibutylzinn(IV)dilaurat oder Molybdänglykolat.

Einkomponenten-Polyurethanlacke, die als Bindemittel und insbesondere Zweikomponenten-Polyurethanlacke, die als Vernetzter nach dem erfindungsgemäßen Verfahren hergestellte Polyisocyanate enthalten, führen zu harten aber noch elastischen Lackfilmen einer hohen Abriebfestigkeit bei ausgezeichneter Haftung auf den unterschiedlichsten Substraten. Darüber hinaus zeichnen sich die Lackfilme durch ihre sehr geringe Verfärbung aus, so dass auch die Maserung damit lackierter heller Holzarten deutlich zur Geltung kommen kann.

Die Polyurethanlacke, die nach dem erfindungsgemäßen Verfahren hergestellte Polyisocyanate als Bindemittel bzw. als Härter enthalten, können selbstverständlich die aus der Lacktechnologie üblichen Hilfs- und Zusatzmittel wie Pigmente, Verlaufshilfsmittel, Füllstoffe u. dergleichen enthalten.

### Beispiele

In den nachfolgenden Beispielen beziehen sich alle Prozentangaben auf das Gewicht. Zur Charakterisierung der erhaltenen Produkte wurden folgende Methoden verwendet:
Die Ermittlung des NCO-Gehaltes der in den Beispielen und Vergleichsbeispielen beschriebenen Harze erfolgte durch Titration gemäß DIN EN ISO 11 909.

Die dynamischen Viskositäten wurden nach DIN 3219 mit einem DIN Messkörper 125 bei 23 °C mit dem Viskosimeter Reolab QC der Fa. Anton Paar im Schergeschwindigkeitsbereich von 1 bis 1600 1/s gemessen.

Die Bestimmung der Restmonomergehalte erfolgte gaschromatographisch gemäß DIN EN ISO 10283.

Der Festtkörpergehalt (nicht verdampfbarer Anteil) wurde nach DIN 3251 unter den dort für Isocyanate beschriebenen Prüfbedingungen durchgeführt.

Die Farbzahlen wurden auf Basis der DIN EN 1557 mit einem LICO 400 der Firma HACH Lange in 50 mm Einweg-Rechteckküvetten bei 23°C gemessen.

### Beispiel 1 (erfindungsgemäß)

In einem 1000ml Doppelmantelplanschliffgefäß werden 660g Toluylendiisocyanat mit einem 2,4-Anteil von rund 80 Gew.-% und einem Gehalt an CIMCH von 3 Gew.-ppm vorgelegt. Das Reaktionsgefäß wird auf die gewünschte Reaktionstemperatur 85°C erhitzt. Anschließend werden in einem Zeitraum von 30 Minuten 80g des auf 65°C vorgewärmten Polyolgemisches, bestehend aus einer Mischung aus Trimethylolpropan und Diethylenglykol, kontinuierlich zudosiert. Die dabei frei werdende Reaktionswärme wird mit Hilfe eines Kälteumwälzthermostaten sicher abgeführt, so daß man von einer isothermen Fahrweise sprechen kann. Nachfolgend wird die urethangruppenhaltige Rohlösung weitere 45 Minuten bei der gegebenen Temperatur nachgerührt. Die anschließend durchgeführte Bestimmung des NCO-Gehaltes ergibt 33,4 Gew.-%, was dem theoretischen Wert entspricht. Die erhaltene viskose farblose Lösung wird durch eine zweistufige Vakuumdünnschichtdestillation (145°C/155°C; p ≤ 0,5mbar) innerhalb von 90 Minuten von überschüssigem monomeren Toluylendisocyanat befreit. Noch in warmem Zustand wird das in einer Menge von 290g erhaltende urethangruppenhaltige Polyisocyanat zu 75 Gew.-% in Ethylacetat eingelöst. Das auf diese Weise erhaltene Lösungsmittel und Urethangruppen haltige Polyisocyanat weist folgende Kennzahlen auf:
NCO-Gehalt = 13,27 Gew.-%
Viskosität = 1490mPa*s @23°C
Restmonomergehalt = 0,29 Gew.-%
Festkörperanteil = 75,2 Gew.-%
APHA-Farbzahl = 25 Hazen

### Beispiel 2 (erfindungsgemäß)

Man verfährt analog zu Beispiel, 1 führt die Reaktion jedoch mit Toluylendiisocyanat mit einem 2,4-Anteil von rund 80 Gew.-% durch, welches durch Gasphasenphosgenierung hergestellt wurde und einen Gehalt an CIMCH unterhalb der Nachweisgrenze (<1Gew.-ppm) aufweist. Das so erhaltene Lösungsmittel und Urethangruppen haltige Polyisocyanat weist folgende Kennzahlen auf:
NCO-Gehalt = 13,24 Gew.-%
Viskosität = 1505mPa*s @23°C
Restmonomergehalt = 0,25 Gew.-%
Festkörperanteil = 74,9 Gew.-%
APHA-Farbzahl = 21 Hazen

### Beispiel 3 (nicht erfindungsgemäß)

Man verfährt analog zu Beispiel 1, führt die Reaktion jedoch mit Toluylendiisocyanat mit einem 2,4-Anteil von rund 80 Gew.-% Qualität durch, welches einen Gehalt an CIMCH von 160 Gew.-ppm aufweist. Das so erhaltene Lösungsmittel und Urethangruppen haltige Polyisocyanat weist folgende Kennzahlen auf:
NCO-Gehalt = 13,23 Gew.-%
Viskosität = 1510mPa*s @23°C
Restmonomergehalt = 0,26 Gew.%
Festkörperanteil = 75,3 Gew.-%
APHA-Farbzahl = 80 Hazen

### Beispiel 4 (nicht erfindungsgemäß)

Man verfährt analog zu Beispiel 1, führt die Reaktion jedoch mit Toluylendiisocyanat mit einem 2,4-Anteil von rund 80 Gew.-% durch, welches einen Gehalt an CIMCH von 70 Gew.-ppm aufweist. Das so erhaltene Lösungsmittel und Urethangruppen haltige Polyisocyanat weist folgende Kennzahlen auf:
NCO-Gehalt = 13,28 Gew.-%
Viskosität = 1500mPa*s @23°C
Restmonomergehalt = 0,27 Gew.-%
Festkörperanteil = 75,0 Gew.-%
APHA-Farbzahl = 62 Hazen

### Beispiel 5 (nicht erfindungsgemäß)

Man verfährt analog zu Beispiel 1 führt die Reaktion jedoch mit Toluylendiisocyanat mit einem 2,4-Anteil von rund 80 Gew.-% durch, welches einen Gehalt an CIMCH von 35 Gew.-ppm aufweist durch. Das so erhaltene urethangruppenhaltige Polyisocyanat weist folgende Kennzahlen auf:
NCO - Gehalt = 13,24 Gew,-%
Viskosität = 1480mPa*s @23°C
Restmonomergehalt = 0,28 Gew.-%
Festkörperanteil = 75,3% Gew.-%
APHA-Farbzahl = 55 Hazen

Die Beispiele 1-5 zeigen, dass bei Einsatz von Toluylendiisocyanat mit CIMCH-Gehalten <5 Gew.-ppm farbhelle Polyisocyanate mit APHA-Farbzahlen <50 Hazen erhalten werden.

### Beispiel 6, Verwendung

Die Polyisocyanate aus Beispiel 1 und Beispiel 3 werden jeweils mit einem handelsüblichen Polyesterpolyol (®Desmophen 1300, Handelsprodukt der Bayer AG, Hydroxyl-Gehalt 4 Gew.-%) zu einer Gesamtkonzentration von 40 Gew.-% in Butylacetat im NCO/OH-Verhältnis 1:1 abgemischt. Der anwendungstechnische Vergleich zeigt keinerlei Unterschiede in den lacktechnischen Eigenschaften.

| | | | Beispiel1 | Beispiel 3 |
|---|---|---|---|---|
| Formulierung Probenvorbereitung nach DIN EN ISO 1513 und anschließende Konditionierung nach ISO 3270 | Menge Desmodur | [g] | 25,9 | 25,9 |
| | Typ Desmophen | | 13001X | 13001X |
| | Menge Desmophen | [g] | 43,5 | 43,5 |
| | Lösungsmittel | | Butylacetat | Butylacetat |
| | Menge Lösungsmittel | [g] | 60,7 | 60,7 |
| | Festkörpergehalt | [%] | 40 | 40 |
| | NCO /OH ratio | | 1,0 | 1,0 |
| Auslaufzeit nach DIN 53211 im 4mm-Becher | 0 h | [m'ss"hh] | 11"87 | 11"94 |
| | 8h | [m'ss"hh] | 12"65 | 12"62 |
| | 24h | [m'ss"hh] | 13"78 | 13"66 |
| | 27h | [m'ss"hh] | 14"00 | 14"97 |
| | 30h | [m'ss"hh] | 14"72 | 15"25 |
| | 48h | [m'ss"hh] | 19"69 | 19"06 |
| | Temperatur | [°C] | 24,8 | 24,8 |
| | Luftfeuchte | [%] | 72 | 72 |
| Pendelhärte nach DIN EN ISO 1522, Pendeldämpfungsprüfung nach König (120µm nass auf Glass) | 7h | [s] | 82 | 82 |
| | 1d | [s] | 157 | 161 |
| | 2d | [s] | 169 | 176 |
| | 3d | [s] | - | - |
| | 7d | [s] | 189 | 190 |
| | Temperatur | [°C] | 23 | 23 |
| | Luftfeuchte | [%] | 55 | 55 |
| Trocknungszeit Bestimmung des Trocknungsgrades von Beschichtungen nach DIN 53150 | Staub Trocken | [min.] | 14 | 14 |
| | Klebfrei | [Std.] | 2 | 2 |
| | Finger Tocken | [Std.] | 5 | 5 |
| | Temperatur | [°C] | 24,8 | 24,8 |
| | Luftfeuchte | [%] | 72 | 72 |
| Kratzfestigkeit nach DIN EN ISO 20566 | 1 Tag | [0-5] 0 gut | 1 | 1 |
| | 7 Tage | [0-5] 5 schlecht | 1 | 1 |
| Beständigkeit gegen Flüssigkeiten nach DIN EN ISO 2812-4 nach 24 h | 1 min. Aceton | [0-5] 0 gut | 5 | 5 |
| | 1 min. BA / EA*) (1:1) | [0-5] 5 schlecht | 4 | 4 |
| Beständigkeit gegen Flüssigkeiten nach DIN EN ISO 2812-4 nach 7 d | 1 min. Aceton | [0-5] 0 gut | 5 | 5 |
| | 1 min. BA / EA*) (1:1) | [0-5] 5 schlecht | 4 | 4 |

| | | | | |
|---|---|---|---|---|
| *): BA / EA: Butylacetat / Ethylacetat | | | | |

## Patentansprüche

1. Verfahren zur Herstellung von Urethangruppen enthaltenden Polyisocyanaten mit APHA-Farbzahlen <50 Hazen durch Umsetzung von zwei- bis vierwertigen organischen Polyhydroxyverbindungen mit überschüssigen Mengen an Toluylendiisocyanat und anschließender destillativer Entfernung von nicht umgesetztem Überschuss an Toluylendiisocyanat auf Restgehalte <0,5 Gew.-%, **dadurch gekennzeichnet, dass** das eingesetzte Toluylendiisocyanat einen Gehalt an 2-Chloro-6-isocyanato-methylcyclohexadienen <5 Gew.-ppm aufweist.

2. Verfahren nach Anspruch 1, **dadurch gekennzeichnet, dass** das eingesetzte Toluylendiisocyanat durch Gasphasenphosgenierung hergestellt wurde.

3. Verfahren nach Anspruch 1 und 2, **dadurch gekennzeichnet, dass** das eingesetzte Toluylendiisocyanat einen Gehalt an 2-Chloro-6-isocyanato-methylcyclohexadienen von unterhalb der Nachweisgrenze von 1 Gew.-ppm aufweist.

4. Verfahren nach Anspruch 1 bis 3, **dadurch gekennzeichnet, dass** die Urethangruppen aufweisenden Polyisocyanate APHA-Farbzahlen <30 Hazen, bevorzugt ≤ 25 Hazen aufweisen.

5. Verfahren nach Anspruch 1 bis 4, **dadurch gekennzeichnet, dass** man als Toluylendiisocyanat 2,4-Toluylendiisocyanat oder dessen technische Gemische mit bis zu 35 Gew.-% bezogen auf Gemisch an 2,6-Toluylendiisocyanat verwendet.

6. Verfahren nach Anspruch 1 bis 5, **dadurch gekennzeichnet, dass** man als Polyhydroxyverbindungen zwei- bis dreiwertige Alkohole mit einem Molekulargewicht von 62 bis 146 und/oder aus ihnen durch Addition von Ethylen- und/oder Propylenoxid hergestellte Polyetherpolyole des Molgewichtsbereichs 106 bis 600 verwendet.

7. Verfahren nach Anspruch 1 bis 5, **dadurch gekennzeichnet, dass** man als Polyhydroxyverbindungen ein Gemisch aus Trimethylolpropan und Diethylenglykol verwendet.

8. Urethangruppen enthaltende Polyisocyanate erhältlich nach einem Verfahren gemäß einem der Ansprüche 1 bis 7.

9. Verwendung der Urethangruppen enthaltenden Polyisocyanate gemäß Anspruch 8 als Polyisocyanatkomponente in Polyurethanlacken.

10. Verwendung gemäß Anspruch 9 als Vernetzer in Zweikomponenten-Polyurethanlacken.

11. Verwendung der Urethangruppen enthaltenden Polyisocyanate, die nach den Verfahren gemäß Anspruch 1 bis 7 hergestellt wurden, als Polyisocyanatkomponente in Polyurethanklebstoffen.

## Claims

1. Method for producing polyisocyanates comprising urethane groups and having APHA colour indices < 50 Hazen by reacting di- to tetra-valent organic polyhydroxy compounds with excess amounts of tolylene diisocyanate and then removing unreacted excess tolylene diisocyanate to residual contents < 0.5 wt.% by distillation, **characterised in that** the tolylene diisocyanate used has a content of 2-chloro-6-isocyanato-methylcyclohexadienes < 5 wt.ppm.

2. Method according to claim 1, **characterised in that** the tolylene diisocyanate used has been produced by gas phase phosgenation.

3. Method according to claim 1 and 2, **characterised in that** the tolylene diisocyanate used has a content of 2-chloro-6-isocyanato-methylcyclohexadienes below the detection limit of 1 wt.ppm.

4. Method according to claim 1 to 3, **characterised in that** the polyisocyanates comprising urethane groups have APHA colour indices < 30 Hazen, preferably ≤ 25 Hazen.

5. Method according to claim 1 to 4, **characterised in that** there is used as the tolylene diisocyanate 2,4-tolylene diisocyanate or commercial mixtures thereof with up to 35 wt.%, based on the mixture, of 2,6-tolylene diisocyanate.

6. Method according to claim 1 to 5, **characterised in that** there are used as the polyhydroxy compounds di- to tetra-hydric alcohols having a molecular weight of from 62 to 146 and/or polyether polyols having a molecular weight in the range of from 106 to 600 prepared therefrom by addition of ethylene oxide and/or propylene oxide.

7. Method according to claim 1 to 5, **characterised in that** there is used as the polyhydroxy compounds a mixture of trimethylolpropane and diethylene glycol.

8. Polyisocyanates comprising urethane groups, obtainable by a method according to any one of claims 1 to 7.

9. Use of the polyisocyanates comprising urethane groups according to claim 8 as the polyisocyanate component in polyurethane coatings.

10. Use according to claim 9 as the crosslinker in two-component polyurethane coatings.

11. Use of the polyisocyanates comprising urethane groups produced by the methods according to claim 1 to 7 as the polyisocyanate component in polyurethane adhesives.

## Revendications

1. Procédé de fabrication de polyisocyanates contenant des groupes uréthane ayant des indices de couleur APHA < 50 Hazen par mise en réaction de composés polyhydroxy organiques bi- à tétravalents avec des quantités en excès de diisocyanate de toluylène puis élimination par distillation de l'excès non réagi de diisocyanate de toluylène à des teneurs résiduelles < 0,5 % en poids, **caractérisé en ce que** le diisocyanate de toluylène utilisé présente une teneur en 2-chloro-6-isocyanato-méthylcyclohexadiènes < 5 ppm en poids.

2. Procédé selon la revendication 1, **caractérisé en ce que** le diisocyanate de toluylène utilisé a été fabriqué par phosgénation en phase gazeuse.

3. Procédé selon les revendications 1 et 2, **caractérisé en ce que** le diisocyanate de toluylène utilisé présente une teneur en 2-chloro-6-isocyanato-méthylcyclohexadiènes inférieure à la limite de détection de 1 ppm en poids.

4. Procédé selon les revendications 1 à 3, **caractérisé en ce que** les polyisocyanates comprenant des groupes uréthane présentent des indices de couleur APHA < 30 Hazen, de préférence ≤ 25 Hazen.

5. Procédé selon les revendications 1 à 4, **caractérisé en ce que** du diisocyanate de 2,4-toluylène ou ses mélanges techniques avec jusqu'à 35 % en poids de diisocyanate de 2,6-toluylène, par rapport au mélange, sont utilisés en tant que diisocyanate de toluylène.

6. Procédé selon les revendications 1 à 5, **caractérisé en ce que** des alcools bi- à trivalents ayant un poids moléculaire de 62 à 146 et/ou des polyéther-polyols fabriqués à partir de ceux-ci par addition d'oxyde d'éthylène et/ou de propylène d'une plage de poids moléculaire de 106 à 600 sont utilisés en tant que composés polyhydroxy.

7. Procédé selon les revendications 1 à 5, **caractérisé en ce qu'**un mélange de triméthylolpropane et de diéthylène glycol est utilisé en tant que composés polyhydroxy.

8. Polyisocyanates contenant des groupes uréthane pouvant être obtenus par un procédé selon l'une quelconque des revendications 1 à 7.

9. Utilisation des polyisocyanates contenant des groupes uréthane selon la revendication 8 en tant que composant polyisocyanate dans des vernis à base de polyuréthane.

10. Utilisation selon la revendication 9 en tant qu'agent de réticulation dans des vernis bicomposants à base de polyuréthane.

11. Utilisation des polyisocyanates contenant des groupes uréthane qui ont été fabriqués par le procédé selon les revendications 1 à 7 en tant que composant polyisocyanate dans des adhésifs à base de polyuréthane.
